# EUROPÄISCHE PATENTANMELDUNG

(11) **EP 2 462 976 A1**
(43) Veröffentlichungstag der Anmeldung: **13.06.2012**
(21) Anmeldenummer: 11189693.2
(22) Anmeldetag: 18.11.2011
(51) Int. Cl.: A61M 25/01, A61M 25/10, A61B 17/00, A61M 39/06, A61N 1/00

(54) **Hochdruckdichte Gleitlagereinrichtung für minimalinvasive Instrumente**

(30) Priorität: 08.12.2010 US 420802 P
(71) Anmelder: Biotronik AG, 8180 Bülach (CH)
(72) Erfinder: Wesselmann, Matthias, 8455 Rüdlingen (CH)
(74) Vertreter: Lindner-Vogt, Karin L.

(57) **Zusammenfassung**

Eine hochdruckdichte Gleitlagereinrichtung für minimalinvasive Instrumente, umfasst
- ein inneres Schaftteil (2),
- ein darauf sitzendes, relativ dazu verschiebbares Außenteil (3), und
- eine gleitfähige Dichtung (5) in einem Spalt (4) zwischen Schaft- und Außenteil (2, 3), wobei die Dichtung (5) aus einem quellfähigen, hydrophilen Material besteht, das durch Einfluss einer Flüssigkeit derart aktivierbar ist, dass durch die Aktivierung der Spalt (4) unter gleichzeitiger Bereitstellung einer Gleitfähigkeit zwischen Schaft- und Außenteil (2, 3) druckdicht verschlossen ist.

## Beschreibung

Die Erfindung betrifft eine hochdruckdichte Gleitlagereinrichtung für minimalinvasive Instrumente, die ein inneres Schaftteil, ein darauf sitzendes, relativ dazu verschiebbares Außenteil und eine gleitfähige Dichtung in einem Spalt zwischen Schaft- und Außenteil aufweist.

Als Beispiel für solche Gleitlagereinrichtungen für minimalinvasive Instrumente sind abgedichtete Gleitverbindungen zwischen einem Innenschaft eines Ballonkatheters und dem Hals eines außenseitig darauf sitzenden Ballons etwa zum Ausbringen eines Stents oder zwischen einem relativ zum Außenschaft beweglichen Innenschaft bei sogenannten Rollmembran-Kathetern zu nennen.

Die in ihrem Offenbarungsgehalt hinsichtlich der vorliegenden Erfindung übereinstimmenden Druckschriften WO 95/11055 A1 und US 2003/0212447 A1 offenbaren eine gleitfähige Dichtung - im vorliegenden kurz "Gleitdichtung" bezeichnet -, die zwischen Gleithülsen und dem Katheter, auf dem sie sitzen, gebildet ist. Die Gleitdichtung ist dabei als Toleranzpassung zwischen dem Innendurchmesser der Gleithülse und dem Außendurchmesser des Katheterschaftes gebildet. In den Druckschriften werden Probleme beim Zurückziehen der Gleithülsen unter Anwendung eines hohen Druckes beschrieben. In diesem Fall kann der Druck so hoch werden, dass sich die Dichtung öffnet und Druckflüssigkeit zwischen dem Innendurchmesser der Gleitdichtung und dem Außendurchmesser des Katheterschaftes austreten kann. Zur Lösung dieses Problems wird in diesen Druckschriften vorgeschlagen, ein zusätzliches Dichtteil an dem Innendurchmesser der Gleithülsen anzukleben, das sich längs der Hülse darunter erstreckt. Diese Dichtanordnung wird daher als "L-Dichtung" bezeichnet. Bei einer Druckerhöhung wird die L-Dichtung enger gegen den Außendurchmesser des Katheters gepresst, was das Risiko einer Flüssigkeitsleckage vermindert. Irgendwelche Maßnahmen zur Erhöhung der Gleitfähigkeit der Dichtung für eine leichtgängige Verschiebung der Hülsen sind dieser Druckschrift nicht entnehmbar. Als Materialien für die Gleithülsen werden lediglich hydrophile Polymere genannt, die gute Gleiteigenschaften haben.

Die US 6,238,410 B1 zeigt eine Katheteranordnung, bei der eine abgedichtete Kammer zwischen einer Innen- und Außenwand einer zurückziehbaren Hülle am distalen Ende eines Katheters gebildet ist. Diese Hülse dient beispielsweise zur Einhüllung eines Stents, der im Bereich einer Stenose in einem Herzgefäß ausgebracht werden soll. Durch das Zurückziehen der Hülle wird der Stent an der gewünschten Position im Gefäß freigesetzt. Die vorgenannte Druckschrift offenbart eine Beschichtung oder Füllung der abgedichteten Kammer mit einer Gleitsubstanz, um die Reibung zwischen der Innen- und Außenwand der Hülle bei deren Zurückziehen zu vermindern, ohne dass ein Kontakt zwischen der Gleitsubstanz und einer Körperflüssigkeit stattfindet. Geeignete Gleitmittel sind beispielsweise Silicone, PVP und PPO. Eine Abdichtung spielt bei der aus der US 6,238,410 B1 bekannten Katheteranordnung keine Rolle, da die Kammer in keinem Fluidkontakt mit einer Druckflüssigkeit steht, die bei diesem Katheter zum Einsatz käme.

Aus der US 5,957,930 A ist ein Katheter zum Ausbringen eines Stents mit einer Schaftdichtung bekannt, die als Gleitdichtung mit dem distalen Ende des Außenschaftes des Katheters verbunden ist und eine gleitende Abdichtung zu dem sich weiter in distaler Richtung erstreckenden Innenschaft bildet. Die Gleitdichtung soll aus einem ionomeren Polyolefin-Copolymer hergestellt sein. Dieses Material hat keine signifikanten Quelleigenschaften, um größere Toleranzen im Bereich der Dichtung überbrücken zu können.

Zusammenfassend sind die aus dem Stand der Technik bekannten Gleitlagereinrichtungen an minimalinvasiven Instrumenten dahingehend nachteilig, dass ihre gleitfähigen Dichtungen auf eine enge Toleranz zwischen Gleitflächen angewiesen sind, um eine genügende Dichtigkeit zu erzielen. Ist die Toleranz zu gering, besteht die Gefahr, dass der Gleitsitz klemmt und damit eine Bewegung der relativ zueinander verschiebbaren Teile nicht mehr oder nur noch schwergängig von statten gehen kann. Ist die Toleranz zu groß, besteht das Risiko einer Leckage im Bereich der gleitfähigen Dichtung. Erschwerend kommt bei den hier in Rede stehenden minimal invasiven Instrumenten hinzu, dass die beteiligten Dichtungspartner oftmals durch Kunststoffe gebildet sind, die durch Relaxation oder Lösungsmittelaufnahme Dimensionsänderungen unterworfen sind. Auch ist eine Aufweitung unter Belastung zum Beispiel durch einen hohen Druck im Katheterschaft - auch bezeichnet als "Compliance" - bei solchen Gleitlagereinrichtungen nach dem Stand der Technik zu beobachten.

Ausgehend von den geschilderten Problemen des Standes der Technik liegt der Erfindung die Aufgabe zugrunde, eine hochdruckdichte Gleitlagereinrichtung für minimal invasive Instrumente anzugeben, bei der die abzudichtenden Spalte zwischen den beteiligten Innen-und Außenteilen der Gleitlagereinrichtung ohne besondere Anforderungen an die Bauteiltoleranzen druckdicht geschlossen werden, gleichzeitig jedoch die Reibung zwischen den Gleitlagerpartnern vermindert wird.

Diese Aufgabe wird durch eine Ausbildung der gleitfähigen Dichtung aus einem quellfähigen, hydrophilen Material gelöst, das durch Einfluss einer Flüssigkeit derart aktivierbar ist, dass durch die Aktivierung der Spalt unter gleichzeitiger Bereitstellung einer Gleitfähigkeit zwischen Schaft und Außenteil druckdicht verschlossen ist.

Vorteilhafterweise sorgt bei dieser erfindungsgemäßen Dichtung das Quellen des Materials für eine zuverlässige, druckdichte Verschließung des abzudichtenden Spaltes, wobei die geringe Reibung mit der Aktivierung der Dichtung vorzugsweise durch Wasser oder einem Kontrastmittel eintritt.

Bevorzugtermaßen besteht die Dichtung aus einer Beschichtung auf Innen- oder Außenteil, die beispielsweise aus einem stark quellfähigen, hydrophilen Material gebildet ist. Es handelt sich z. B. um ein Hydrogel auf Basis von Polyvinylpyrrolidone. Andere als Beschichtung nutzbare Hydrogele können ebenfalls verwendet werden, wenn sie genügend stark quellen, um den Spalt zu überbrücken, und gute Reibeigenschaften bieten.

Bevorzugte Ausführungsformen sehen eine Anbringung der Beschichtung auf der Außenseite des inneren Schaftteils oder der Innenseite des Außenteils auf. Die jeweilige Variante ist nach den Gegebenheiten bei der Dichtung auszuwählen. Dabei ist darauf zu achten, dass bei solchen Anwendungen, bei denen das Blockieren der Gleitdichtung mit besonders negativen Auswirkungen auf den Patienten oder großen Risiken verbunden sind, der die Dichtung an sich bereitstellende Bereich einer besonders dicken Beschichtung in längsaxialer Richtung kurz und die Dichtwirkung im gesamten Gleitbereich durch das darauf gleitende Element sichergestellt wird. Letzteres kann in hoher dimensionaler Präzision hergestellt werden. Insoweit ist es von Vorteil, wenn die Beschichtung auf dem inneren Schaftteil eine Gleitfläche für das relativ verschiebbare Außenteil mit einer axialen Länge bildet, die größer als die axiale Länge einer am Außenteil gebildeten Gleitdichtfläche ist. Vorzugsweise ist die Länge der Gleitfläche mehr als doppelt so groß wie die axiale Länge der Gleitdichtfläche.

Bevorzugte Anwendungen der erfindungsgemäßen Gleitlagereinrichtung betreffen eine Gleitdichtung zwischen einem Innenschaft und dem Hals eines dilatierbaren Ballons eines Ballonkatheters bzw. zwischen dem Innenschaft und Außenschaft eines Katheters, wobei die Dichtung durch eine Beschichtung im Bereich des distalen Endes des Außenschaftes gebildet ist. Vorzugsweise sitzt dann die Beschichtung auf der Außenseite des Innenschaftes der Gleitlagereinrichtung.

Weitere Merkmale, Einzelheiten und Vorteile der Erfindung ergeben sich aus der nachfolgenden Beschreibung von Ausführungsbeispielen anhand der beigefügten Zeichnungen. Es zeigen:
- Fig. 1 und 2: schematische Längsaxialschnitte einer Gleitlagereinrichtung mit einem Innenschaft und einem Ballon vor und nach Auslängung des Ballons,
- Fig. 3 und 4: schematische Längsaxialschnitte analog Fig. 1 und 2 in einer zweiten Ausführungsform der Gleitlagereinrichtung, und
- Fig. 5 und 6: schematische Längsaxialschnitte einer Gleitlagereinrichtung mit einem Innen- und Außenschaft eines Katheters in zwei unterschiedlichen Relativpositionen von Innen- zu Außenschaft.

Die in den Figuren gezeigten Gleitlagereinrichtungen 1 dienen zur Anwendung bei minimalinvasiven Instrumenten. Sie haben gemeinsam ein inneres Schaftteil 2 und ein darauf sitzendes, relativ dazu verschiebbares Außenteil 3. Im Spalt 4 zwischen den Komponenten sitzt eine gleitfähige Dichtung 5, die aus einem stark quellfähigen, hydrophilen Material besteht. Wie noch näher zu erläutern ist, wird dieses Material durch Einfluss einer Flüssigkeit aktiviert und schwillt damit auf. Damit wird der Spalt 4 zwischen dem inneren Schaftteil 2 und dem Außenteil 3 hochdruckfest abgedichtet. Gleichzeitig wird durch die Hydrophilität des Materials der Dichtung 5 eine gute Gleitfähigkeit zwischen innerem Schaftteil 2 und Außenteil 3 erzielt.

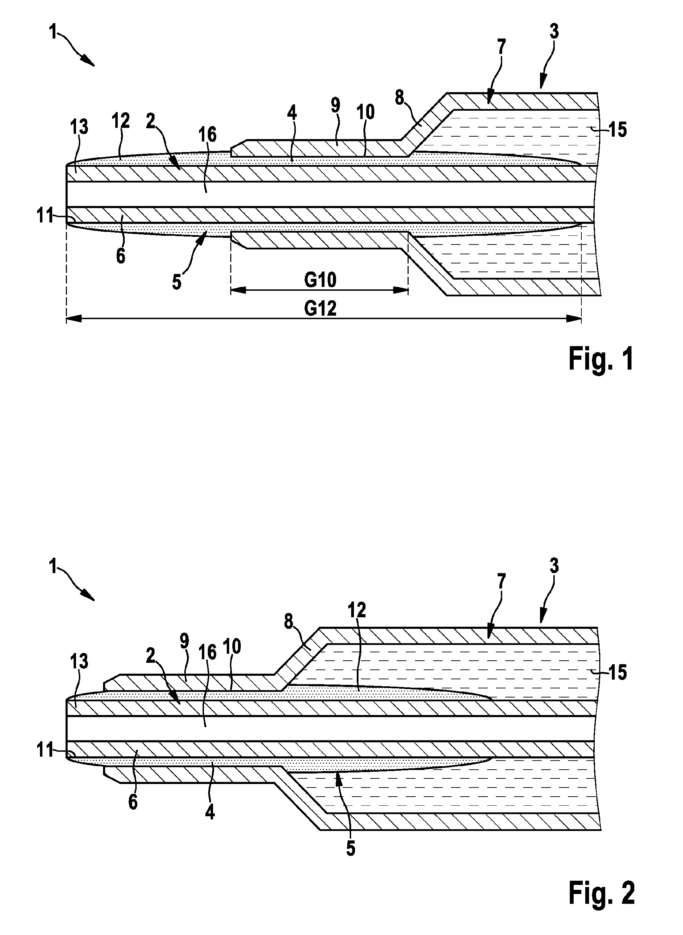

Bei dem in Fig. 1 und 2 gezeigten Ausführungsbeispiel ist das innere Schaftteil 2 durch den Innenschaft 6 eines minimalinvasiven Instrumentes in Form eines Ballonkatheters gebildet. In diesen Zeichnungen ist der dilatierbare Ballon 7 als relativ zum Innenschaft 6 verschiebbares Außenteil dargestellt. Der Ballon 7 geht über eine Verjüngung 8 in den distalen Ballonhals 9 über, dessen zylindermantelförmige Innenseite eine Gleitdichtfläche 10 zum Innenschaft 6 hin bildet. Die Dichtung 5 selbst ist durch ein Hydrogel auf der Außenseite 11 des Innenschaftes 6 aufgebrachte Beschichtung 12 ausgebildet, die sich über eine axiale Gleitlänge G12 vom distalen Ende 13 des Innenschaftes aus in proximaler Richtung erstreckt. Die Gleitlänge G12 der Beschichtung 12 ist dabei gegenüber der Länge G10 der Gleitdichtfläche 10 am Ballonhals 9 deutlich länger, nämlich vorzugsweise mehr als das Doppelte.

Wie aus einem Vergleich der Fig. 1 und 2 hervorgeht, ist damit eine vorteilhafte schwimmende Anbindung des distalen Endes des Ballons 7 in Form seines Ballonhalses 9 an den Innenschaft 6 möglich. Dies ist besonders vorteilhaft bei der Anwendung des gezeigten Erfindungsgegenstandes bei langen Angioplastieballonen mit unterschiedlichem Druckansprechen - der sogenannten "Compliance" - von Ballon und Innenschaft. Eine unterschiedliche plastische Längenausdehnung von Schaft und Ballon unter Druck kann bei fest angebundenen Ballonen zu Problemen führen. So ist bei einer zu großen Längenausdehnung des Ballons gegenüber dem Innenschaft mit Querfalten des Ballons nach dem Deflatieren und damit zu erhöhten Rückzugskräften beim Entfernen des Katheters zu rechnen. Wird der Innenschaft nach Inflatieren und Deflatieren des Ballons zu lang im Vergleich zum Ballon, kann der verwendete Führungsdraht nach Entfernen kein zweites Mal mehr eingeführt werden, da sich der Innenschaft beim Wiedereinführen des Drahtes zunächst aufstaucht und schließlich abknickt.

In beiden Fällen hilft die schwimmende Anbindung des Ballonendes, wie ein Vergleich der Fig. 1 und 2 zeigt. So wird vor dem in Fig. 1 gezeigten Zustand die Beschichtung 12 durch den Einfluss des in den Ballon 7 eingeführten Druckfluids aktiviert, das heißt, die vorher relativ dünne Beschichtung 12 quillt deutlich in die in Fig. 1 gezeigte Konfiguration auf, wo sie einerseits eine hochdruckfeste Dichtung im Spalt 4 zwischen Ballonshals 9 und Innenschaft 6 bereitstellt. Diese Dichtung wurde bis zu 30 bar erfolgreich bei Entwicklungsarbeiten zu der vorliegenden Erfindung getestet. In keinem, Fall versagte der Ballon oder die Dichtung. Der Maximaldruck, dem eine derartige Dichtung widersteht, lässt sich entsprechend leicht durch Anpassen der Dichtungslänge erhöhen. Durch die Hydrophilität des Dichtungsmaterials kann sich der Ballonhals 9 trotzdem leichtgängig auf der Beschichtung 12 verschieben und damit bei einer Auslängung in Richtung zum distalen Ende 12 auf der Beschichtung um die Länge ΔL verschieben. Damit werden Querfalten oder ein Abknicken des Innenschaftes 6 bei unterschiedlichen Ausdehnungen von Ballon 7 und Innenschaft 6 vermieden. Der Innenschaft 6 ist spannungsfrei, ohne dass das Material des Ballons 7 und des Innenschaftes 6 in ihrer Compliance aufeinander abgestimmt sein müssen.

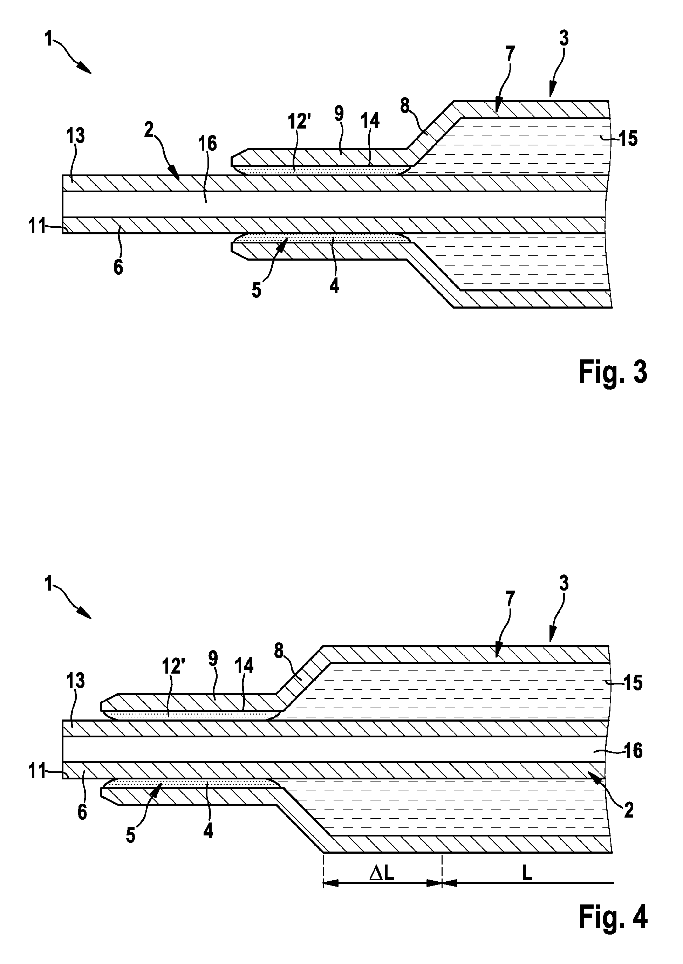

Die in den Fig. 3 und 4 gezeigte Ausführungsform einer Gleitlagereinrichtung ist wiederum zwischen einem Innenschaft 6 und dem Ballonhals 9 eines Ballons 7 angelegt. Im Unterschied zur Ausführungsform gemäß Fig. 1 und 2 ist hier die Beschichtung 12' entsprechenden Materials auf der Innenseite 14 des Ballonshalses 9 aufgebracht. Die Abdichtung erfolgt in gleicher Weise, wie sie anhand von Fig. 1 und 2 bereits beschrieben wurde. Bei der Auslängung des Ballons um das Maß ΔL seiner Länge L nach dem Aufquellen der Beschichtung 12' wiederum durch Aktivierung mittels des Druckfluids 15 herrscht eine gleichmäßig kleine Reibung beim Gleiten des Ballonhalses 9 auf dem Innenschaft 6.

Mit Hilfe der in Fig. 3 und 4 gezeigten Konstruktion ist es auch möglich, einen Katheter ohne Innenschaft 6 zu realisieren. Herkömmlicherweise wird in dem Lumen 16 im Innenschaft 6 ein Führungsdraht (nicht gezeigt) positioniert, auf dem der Katheter zu seinem Bestimmungsort in einem Körpergefäß weitergeschoben wird. Bei der weiteren, hier nicht eigens dargestellten Neuerung wird vielmehr der Innenschaft 6 durch den Führungsdraht selbst realisiert, mit dessen Außenseite dann der Ballonhals 9 über seine Beschichtung 12' abdichtet und gleichzeitig eine leichtgängige Verschiebbarkeit dieser beiden Komponenten zueinander gewährleistet wird. Der Führungsdraht selbst muss dabei fluiddicht sein und sein Außendurchmesser muss auf den Dichtungsdurchmesser des Katheters abgestimmt sein. Durch diese Konstruktion können das Profil des minimalinvasiven Instruments weiter im Querschnitt reduziert werden und/oder die Deflationszeiten verbessert werden, da das Ballonlumen um den weggefallenen Querschnitt des Innenschaftes vergrößert werden kann.

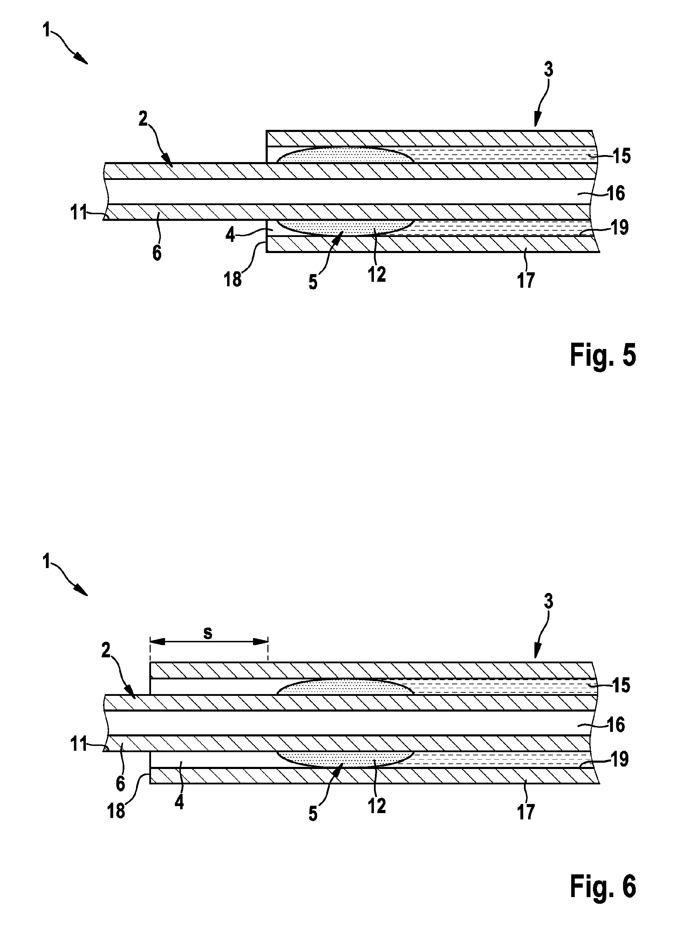

Die in Fig. 5 und 6 dargestellte Version einer Gleitlagereinrichtung 1 ist besonders für sogenannte Rollmembrankatheter bestimmt, bei denen der Innenschaft 6 relativ zu einem Außenschaft 17 bewegt werden muss. Die Gleitlagereinrichtung 1 selbst ist im gezeigten Fall durch eine Beschichtung 12 auf der Außenseite 11 des Innenschaftes 6 gebildet, die bei der in Fig. 5 gezeigten Ausgangsposition der Anordnung vor dem distalen Ende 18 des Außenschaftes 17 sitzt. Die Beschichtung 12 ist wiederum aus dem in Verbindung mit Fig. 1 und 2 erwähnten Material gebildet. Durch Aktivierung mittels des Druckfluids 15 quillt sie auf und stellt zur Innenseite 19 des Außenschaftes 17 eine hochdruckfeste Abdichtung bei gleichzeitiger Gleitlagerung zwischen Innen- 6 und Außenschaft 17 her. Durch diese dichte Gleitlagerung ist der Innenschaft 6 relativ zum Außenschaft 17 leichtgängig verschiebbar. Die in Fig. 6 eingezeichnete Verschiebungslänge s dient beispielsweise zur Steuerung der Rollmembran bei einem Rollmembrankatheter.

Zusammenfassend wird durch den Erfindungsgegenstand eine einfach herstellbare, hochdruckdichte Gleitführung für minimalinvasive Instrumente geschaffen. Die Gleitfunktion wird dabei erst aktiviert, wenn das hydrophile Dichtungsmaterial durch Wasser oder ein Kontrastmittel benetzt wird. Damit können Komponenten von minimalinvasiven Instrumenten fixiert und zu einem vorgegebenen Zeitpunkt beweglich gemacht werden.

## Patentansprüche

1. Hochdruckdichte Gleitlagereinrichtung für minimalinvasive Instrumente, umfassend
- ein inneres Schaftteil (2),
- ein darauf sitzendes, relativ dazu verschiebbares Außenteil (3), und
- eine gleitfähige Dichtung (5) in einem Spalt (4) zwischen Schaft- und Außenteil (2,3),
**dadurch gekennzeichnet, dass**
- die Dichtung (5) aus einem quellfähigen, hydrophilen Material besteht, das durch Einfluss einer Flüssigkeit derart aktivierbar ist, dass durch die Aktivierung der Spalt (4) unter gleichzeitiger Bereitstellung einer Gleitfähigkeit zwischen Schaft- und Außenteil (2, 3) druckdicht verschlossen ist.

2. Gleitlagereinrichtung nach Anspruch 1, **dadurch gekennzeichnet, dass** die Dichtung (5) durch eine Beschichtung (12, 12') gebildet ist.

3. Gleitlagereinrichtung nach Anspruch 1 oder 2, **dadurch gekennzeichnet, dass** die Flüssigkeit zur Aktivierung der Dichtung (5) durch Wasser oder ein Kontrastmittel gebildet ist.

4. Gleitlagereinrichtung nach einem der vorgenannten Ansprüche, **dadurch gekennzeichnet, dass** die Dichtung (5) aus Hydrogelen wie Polyvinylpyrrolidone (PVP), Hyaloronsäure, PEG, Dextran oder vernetztem Zucker besteht.

5. Gleitlagereinrichtung nach Anspruch 2, **dadurch gekennzeichnet, dass** die Beschichtung (12,) auf der Außenseite (11) des inneren Schaftteils (2) aufgebracht ist.

6. Gleitlagereinrichtung nach Anspruch 2, **dadurch gekennzeichnet, dass** die Beschichtung (12') auf der Innenseite (14) des Außenteils (3) aufgebracht ist.

7. Gleitlagereinrichtung nach Anspruch 5, **dadurch gekennzeichnet, dass** die Beschichtung (12) auf dem inneren Schaftteil (2) eine Gleitfläche für das relativ verschiebbare Außenteil (3) mit einer axialen Länge (G12) bildet, die größer als die axiale Länge (G10) einer am Außenteil (3) gebildeten Gleitdichtfläche (10) ist.

8. Gleitlagereinrichtung nach Anspruch 7, **dadurch gekennzeichnet, dass** die axiale Länge (G12) der Gleitfläche mehr als doppelt so groß als die axiale Länge (G10) der Gleitdichtfläche (10) ist.

9. Gleitlagereinrichtung nach einem der vorgenannten Ansprüche, wobei das Außenteil (3) durch einen Hals (9) eines dilatierbaren Ballons (7) eines Ballonkatheters gebildet ist, **dadurch gekennzeichnet, dass** die Dichtung (5) auf der Innenseite (14) des Ballonhalses (9) als Beschichtung (12') aufgebracht ist.

10. Gleitlagereinrichtung nach Anspruch 9, **dadurch gekennzeichnet, dass** der Ballonhals (9) über die Dichtung (5) schwimmend auf dem inneren Schaftteil (2) gelagert ist.

11. Gleitlagereinrichtung nach einem der vorgenannten Ansprüche, wobei das innere Schaftteil (2) durch einen Innenschaft (6) und das Außenteil (3) durch einen Außenschaft (17) eines Katheters gebildet sind, **dadurch gekennzeichnet, dass** die Dichtung (5) durch eine Beschichtung (12) im Bereich des distalen Endes (18) des Außenschaftes (17) gebildet ist.

12. Gleitlagereinrichtung nach Anspruch 11, **dadurch gekennzeichnet, dass** die Beschichtung (12) auf die Außenseite (11) des Innenschaftes (6) aufgebracht ist.
